# EUROPEAN PATENT APPLICATION

(11) **EP 4 559 380 A1**
(43) Date of publication of application: **28.05.2025**
(21) Application number: 23212342.2
(22) Date of filing: 27.11.2023
(51) Int. Cl.: A61B 5/00

(54) **PLAQUE DETECTION DEVICE FOR THE DETECTION OF PLAQUE ON A SURFACE OF A TOOTH**

(71) Applicant: Koninklijke Philips N.V., 5656 AG Eindhoven (NL)
(72) Inventor: GOTTENBOS, Bart, Eindhoven (NL)
(74) Representative: Philips Intellectual Property & Standards

(57) **Abstract**

The present invention relates to a plaque detection device for the detection of plaque on a surface of a tooth. The plaque detection device comprises a mechanical probe including a probe tip being configured for moving along a surface of a tooth and comprising a first surface and a second surface, the first surface being configured for moving along the surface of the tooth, and the second surface being directed away from the surface of the tooth. The plaque detection device comprises further an optical sensor, the optical sensor being directed towards the second surface of the probe tip and configured for detecting light that is reflected and/or emitted from the second surface of the probe tip. The plaque detection device comprises further a processing unit, the processing unit being configured for receiving a signal of the optical sensor indicative of the light that is reflected and/or emitted from the second surface of the probe tip, and configured for determining a presence of plaque on the surface of the tooth based on a variation of the light that is reflected and/or from the second surface of the probe tip.

## Description

### FIELD OF THE INVENTION

The present invention relates to a plaque detection device for the detection of plaque on a surface of a tooth, an oral care apparatus comprising the plaque detection device, and a method for the detection of plaque on a surface of a tooth using the plaque detection device.

### BACKGROUND OF THE INVENTION

Dental plaque detection is one of the key needs in oral healthcare. Plaque detection can be a break-through feature for oral healthcare, since it enables feedback to the user of a tooth brush where to brush better or longer. Several technologies are known to detect plaque, which may have many disadvantages and are not easily applicable. For example, the techniques of disclosing stain or fluorescent disclosing stain, together with an examination of the teeth after disclosing the stain using a mirror or a camera, requires an extra step after brushing of the teeth as well as additional consumables. In addition, stains may remain temporarily on the teeth. The technique of red auto-fluorescent plaque imaging requires illumination with blue light, a filter, a camera and image analysis, but discloses only a small fraction of the plaque, and not even all users have this specific type of plaque. Therefore, reliable and comfortable plaque detection remains a challenging task.

Plaque detection should ideally be performed during brushing of the teeth, which is not satisfactorily solved by any of these technologies. Plaque detection during brushing should solve the following problems: It should work without any contrast agent as users typically do not like the need for an extra consumable to be applied to the teeth, and it should detect all of the plaque, in particular all kinds of plaque and also younger plaque. Preferably, also the location of the plaque should be detected, such that a plaque map or an image can be created.

The inventors of the present invention have thus found that it would be advantageous to have an improved device for the detection of plaque on a surface of a tooth that does not have the above-mentioned drawbacks, and that provides reliable and comfortable plaque detection preferably during brushing of the teeth.

### SUMMARY OF THE INVENTION

It is an object of the present invention to provide an improved plaque detection device that works without any contrast agent, detects all kinds of plaque, and can be performed during brushing of the teeth.

The object of the present invention is solved by the subject matter of the independent claims, wherein further embodiments are incorporated in the dependent claims.

The described embodiments similarly pertain to the plaque detection device for the detection of plaque on a surface of a tooth, the oral care apparatus comprising the plaque detection device, and the method for the detection of plaque on a surface of a tooth using the plaque detection device. The embodiments described further may be combined in any possible way. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method, unless explicitly mentioned to the contrary hereinafter.

According to a first aspect of the invention, there is provided a plaque detection device for the detection of plaque on a surface of a tooth. The plaque detection device comprises a mechanical probe comprising a probe tip being configured for moving along a surface of a tooth, wherein the probe tip comprises a first surface and a second surface, the first surface being configured for moving along the surface of the tooth, and the second surface being directed away from the surface of the tooth. The plaque detection device comprises further an optical sensor, the optical sensor being directed towards the second surface of the probe tip and configured for detecting light that is reflected and/or emitted from the second surface of the probe tip, and a processing unit, the processing unit being configured for receiving a signal of the optical sensor indicative of the light that is reflected and/or emitted from the second surface of the probe tip, and configured for determining a presence of plaque on the surface of the tooth based on a variation of the light that is reflected and/or emitted from the second surface of the probe tip.

Thus, the plaque on a tooth is detected by combining optical sensing or imaging with a mechanical probe ploughing or scratching through the plaque along a surface of a tooth. The mechanical probe comprises a probe tip that moves along the surface of the tooth, when the device is moved relative to the tooth. The probe tip comprises a first surface and a second surface, which can be two portions of a preferably curved outer surface of the probe tip, or the first surface and the second surface can be separated from each other by an edge of the probe tip. Basically, the probe tip is configured such that a portion of the probe tip can be scratched, i.e. moved with direct contact to the tooth, along the surface of the tooth, thereby ploughing through the plaque on the tooth, which can be a soft deposited layer on the tooth. This portion with direct contact to the tooth is regarded as the first surface of the probe tip. When ploughing through the plaque, the probe tip loosens or releases at least a part of the plaque from the tooth, which is then deposited on the probe tip, and preferably on a portion or a surface of the probe tip that is designed for not having direct contact to the tooth. This portion of the probe tip that has no direct contact to the tooth and is configured for being covered with the plaque released from the tooth is regarded as the second surface of the probe tip. As it shall not have contact to the tooth, but shall accumulate the plaque removed from the tooth, the second surface is directed away from the surface of the tooth.

For example, the mechanical probe can have a round probe tip, or it can be an elongated probe with at least a round or oval cross section, like, e.g. a bristle of a tooth brush. In this case, the first surface and the second surface are essentially both part of the same outside surface of the cylindrical probe, with one side of the probe tip contacting the tooth and the other opposite side of the probe being directed away from the tooth. The term of being directed away from the tooth is to be understood such that at least a part of the second surface is visible and not hidden by the tooth when the first surface is in contact with the tooth.

As another example, the probe tip can have a basically flat outer shape, with a lower surface, the first surface, contacting the tooth, and an upper surface, the second surface, being positioned essentially opposite to the first surface. In this case, the first surface and the second surface can be separated from each other by more or less sharp edges of the probe tip.

Thus, while the first surface is facing the tooth surface, the second surface is facing in the other direction and preferably in the direction of the optical sensor. Optionally, the second surface can be directed away from the tooth at a certain angle, that may be, for example, 45 degrees with respect to the surface of the tooth. This may be in particular useful if the optical sensor is positioned not directly above the probe tip with respect to a contact point of the probe tip with the tooth, but slightly further away to the side in a direction parallel to the tooth surface.

The plaque detection device comprises further the optical sensor, which is directed towards the second surface of the probe tip and configured for detecting light that is reaching the optical sensor and coming from the second surface of the probe tip. Thus, the optical sensor can determine a colour or an amount of light being reflected and/or emitted from the second surface of the probe tip. In particular, the optical sensor can be configured for detecting a change in the amount of light being reflected from the second surface of the probe tip. In addition or alternatively, the optical sensor can be configured for detecting a change in the amount of light being emitted from the second surface of the probe tip. Thus, an increasing amount of plaque deposited on the second surface of the probe tip changes an optical property of the second surface and results in a change of the amount of light that is reaching the optical sensor from the direction of the second surface of the probe tip. Light that is emitted from the probe tip can be understood as light that is generated by fluorescence of the probe tip and/or the plaque layer on the probe tip, as well as light that is generated by a luminescent probe tip.

The processing unit is configured for receiving a signal of the optical sensor that is indicative of the light that is reflected and/or emitted from the second surface of the probe tip, and determines a presence of plaque on the surface of the tooth based on a variation of the light that is reflected and/or emitted from the second surface of the probe tip. Thus, preferably, the presence of plaque on the surface of the tooth can be derived from the presence of plaque on the second surface of the probe tip when the probe tip is being moved along the surface of the tooth and loosens plaque from the tooth that is deposited on the probe tip.

Therefore, at an abstract level, the invention combines mechanical manipulation of a surface with optical sensing or imaging in order to get information on the manipulated surface, specifically on the presence or absence of soft, breakable, or loose material on the surface, and may be inspired by the way a dentist detects plaque using a probe.

In an embodiment of the invention, the presence of plaque on the surface of the tooth is determined by the presence of plaque on the second surface of the probe tip when the probe tip is being moved along the surface of the tooth.

In an embodiment of the invention, the probe tip is configured for mechanically scraping plaque off the surface of the tooth and depositing the scraped off plaque on the second surface of the probe tip. Therefore, the probe tip can be shaped such that at least a part of the probe tip penetrates an interspace between the surface of the tooth and a layer of the plaque on the tooth, and removes at least a part of the plaque from the tooth by depositing the removed plaque on the probe tip. For example, the probe tip can have a V-shaped edge between the first surface and the second surface to lift plaque over the edge of the probe tip.

In an embodiment of the invention, the second surface of the probe tip comprises an optical property that is different from an optical property of plaque. The optical property can be a reflectivity of the probe tip that is different from a reflectivity of the plaque. In addition or alternatively, the optical property can be a fluorescence of the probe tip, which differs from a fluorescence of plaque. Further, the optical property can be a luminescence, illumination or illuminance of the probe tip, such that light is coming from the probe itself, which may comprise an integrated light source. All of these differences in at least one optical property between the second surface of the probe tip and the plaque may enhance the contrast between a part of the second surface that is not covered by plaque and a part of the second surface that is covered by plaque and increase the detectability of plaque with the optical sensor. In an embodiment of the invention, the second surface of the probe tip is colored black. Alternatively, the second surface of the probe tip can be colored dark or can comprise at least a dark surface with a low reflectivity. As the plaque usually has a whitish color, this increases the contrast and therefore enhances the detectability of the plaque.

In an embodiment of the invention, the probe tip or at least the second surface of the probe tip is non-fluorescent or has a fluorescence that is different from a fluorescent of plaque. As plaque can have a green or red auto-fluorescence, the second surface preferably has a fluorescence in a different color.

In an embodiment of the invention, the plaque detection device further comprises an illumination source configured for illuminating the second surface of the probe tip with light. This may enhance a homogenous illumination of the second surface of the probe tip and ensure a constant illumination of the probe tip, even in dark environments. In this case, the presence of whitish plaque on a preferably dark probe tip will probably increase the amount of light reflected from the probe tip to the optical sensor.

In addition or alternatively, an illumination source of the plaque detection device can be integrated in the probe tip. Thus, for example, the probe can be or at least comprise a light fiber that emits light at the probe tip thus leading to a luminescent second surface of the probe tip. In this case, the second surface may be at least partially transparent or translucent, such that light coming from the inside of the probe tip can pass the second surface and reach the optical sensor. In this embodiment, the luminescence of the probe tip may be decreased by the presence of plaque covering the second surface.

In an embodiment of the invention, the optical sensor is a camera.

In an embodiment of the invention, the plaque detection device comprises a plurality of mechanical probes. For examples, bristles of a tooth brush can be used as mechanical probe.

In an embodiment of the invention, the mechanical probe comprises a connecting element configured for connecting the probe tip to a body of the plaque detection device.

In an embodiment of the invention, the connecting element is flexible, and/or the connecting element is angled with respect to a longitudinal axis of the plaque detection device. Thus, the probe tip can be connected to a body of the plaque detection device by a connecting element, that can be flexible, i.e. that provides a certain degree of elasticity, in order to ensure a continuous contact of the first surface of the probe tip to the surface of the tooth. Further, the flexible connecting element can ensure that the first surface of the probe tip is contacting the tooth within a predefined range of force. Providing a connecting element that is angled with respect to a longitudinal axis of the plaque detection device can enhance the flexibility of the connecting element with respect to a direction perpendicular to the surface of the tooth.

In an embodiment of the invention, the plaque detection device comprises a removing system configured for removing plaque sticking to the second surface, and/or the second surface of the probe tip is configured for minimizing an amount of plaque sticking to the second surface. Thus, the plaque detection device can provide a fluidic jet for removing the plaque from the second surface of the probe tip, or can utilize vibrations for shaking of the plaque from the probe tip. In addition or alternatively, the second surface of the probe tip can have a very smooth and slippery surface for avoiding plaque sticking to the second surface. Preferably, the second surface can be made of a material or can comprise a material with a low surface energy, like, for example, polytetrafluoroethylene.

In an embodiment of the invention, the plaque detection device further comprises a position sensor configured for determining a position of the plaque detection device with respect to a mouth of a user, and the processing unit is configured for providing a position of the plaque on the surface of the tooth with respect to the mouth of the user. Thus, the plaque detection device can provide a map of the mouth of the user and indicate in which regions of the mouth the teeth are still covered with plaque and should be brushed more carefully.

According to another aspect of the invention, there is provided an oral care apparatus comprising the plaque detection device according to any of the preceding embodiments. Thus, the plaque detection device can be integrated in a toothbrush or any other oral hygiene device like an irrigator. For example, the oral care products having dental plaque detection can be a toothbrush with embedded plaque detection, an irrigator with plaque detection, a mouthpiece with plaque detection, or it can be a separate plaque detection device, for example for use after brushing. Skin care, beauty and shaving products may also use the invention to detect skin deposits or properties.

According to another aspect of the invention, there is provided a method for the detection of plaque on a surface of a tooth using the plaque detection device according to any of the preceding embodiments, the method comprising the steps of providing a plaque detection device according to any of the preceding embodiments, moving the probe tip of the mechanical probe along a surface of a tooth, detecting light that is reflected and/or emitted from the second surface of the probe tip using the optical sensor, and determining a presence of plaque on the surface of the tooth based on a variation of the light that is reflected and/or emitted from the second surface of the probe tip using the processing unit.

In an embodiment of the invention, the method comprises further the step of scraping plaque off the surface of the tooth and depositing the scraped off plaque on the second surface of the probe tip.

In summary, the invention relates to a plaque detection device for the detection of plaque on a surface of a tooth. The plaque detection device comprises a mechanical probe including a probe tip being configured for moving along a surface of a tooth and comprising a first surface and a second surface, the first surface being configured for moving along the surface of the tooth, and the second surface being directed away from the surface of the tooth. The plaque detection device comprises further an optical sensor, the optical sensor being directed towards the second surface of the probe tip and configured for detecting light that is reflected and/or emitted from the second surface of the probe tip. The plaque detection device comprises further a processing unit, the processing unit being configured for receiving a signal of the optical sensor indicative of the light that is reflected and/or emitted from the second surface of the probe tip, and configured for determining a presence of plaque on the surface of the tooth based on a variation of the light that is reflected and/or emitted from the second surface of the probe tip.

One of the advantages of embodiments of the present invention is that plaque can be reliably be detected without the requirement of a contrast agent or a special liquids. Another advantage may reside in that all plaque can be detected new plaque as well as old plaque. Further, when using a camera and an array of mechanical probes, mapping of plaque coverage on teeth can be performed. As future tooth brushes can be equipped with some form of camera for other purposes, such as high resolution location sensing or tooth and gum health sensing, this can be advantageously combined with plaque sensing according to the invention. Another advantage may be that plaque detection can be performed during brushing collecting real-time data, although toothpaste may be a noise factor.

These advantages are non-limiting and other advantages may be envisioned within the context of the present application.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a schematic setup of a plaque detection device for the detection of plaque on a surface of a tooth according to an embodiment of the invention.
Fig. 2 shows a schematic setup of a plaque detection device for the detection of plaque on a surface of a tooth according to another embodiment of the invention.
Fig. 3 shows a photograph of plaque on a probe tip of a plaque detection device according to an embodiment of the invention.
Fig. 4 shows a schematic setup of an oral care apparatus comprising the plaque detection device for the detection of plaque on a surface of a tooth according to an embodiment of the invention.
Fig. 5 shows a block diagram of a method for the detection of plaque on a surface of a tooth using the plaque detection device according to an embodiment of the invention.

### DETAILED DESCRIPTION OF EMBODIMENTS

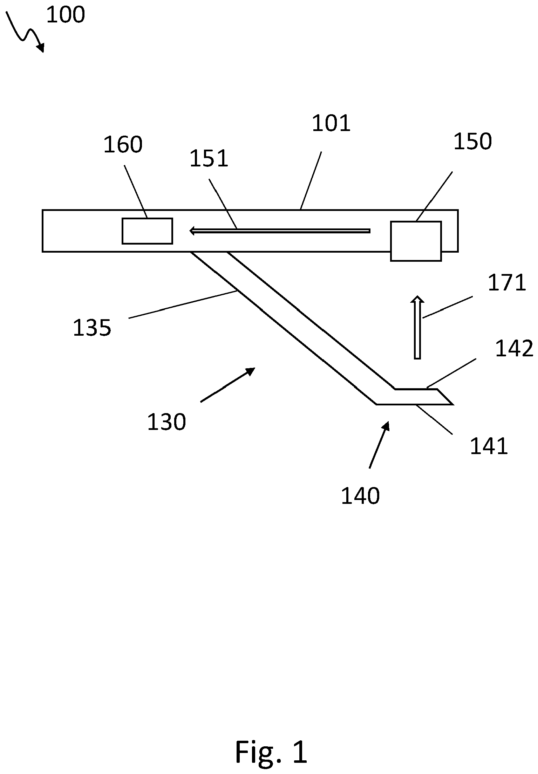

Fig. 1 shows a schematic setup of a plaque detection device 100 for the detection of plaque 110 on a surface of a tooth 120 according to an embodiment of the invention. The plaque detection device 100 comprises a mechanical probe 130 having a probe tip 140, an optical sensor 150, and a processing unit 160. The probe tip 140 has a first surface 141 that is configured for moving along the surface of a tooth 120, and a second surface 142 that is directed away from the surface of the tooth 120. The probe tip 140 of the mechanical probe 130 is connected via a connecting element 135 with a body 101 of the plaque detection device 100. The optical sensor 150 is directed towards the second surface 142 of the probe tip 140 and is configured for detecting light 171 that is reflected and/or emitted from the second surface 142 of the probe tip 140. The processing unit 160 is configured for receiving a signal 151 of the optical sensor 150 that is indicative of the light 171 that is reflected and/or emitted from the second surface 142 of the probe tip 140. Based on a variation of the light 171 that is reflected and/or emitted from the second surface 142 of the probe tip 140, the presence of plaque 110 on the surface of the tooth 120 is determined.

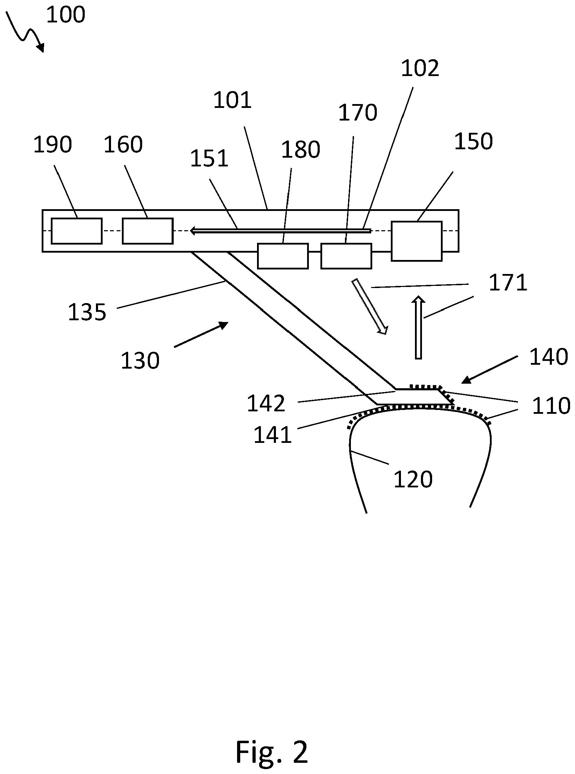

Fig. 2 shows a schematic setup of a plaque detection device 100 for the detection of plaque 110 on a surface of a tooth 120 according to another embodiment of the invention. In this figure a tooth is shown that has a layer of plaque 110 on the surface of the tooth 120. As the mechanical probe 130 with the first surface 141 of the probe tip 140 is ploughing or scratching through the plaque 110 along the surface of the tooth 120 when the plaque detection device 100 is moved relative to the tooth, at least a portion of the plaque 110 is removed from the surface of the tooth 120 and deposited on the second surface 142 of the probe tip 140. The first surface 141 and a second surface 142 can be two portions of a preferably curved outer surface of the probe tip 140, or they can be separated from each other by an edge of the probe tip 140. When ploughing through the plaque 110, the probe tip 140 loosens or releases at least a part of the plaque 110 from the surface of the tooth 120, which is then deposited on the probe tip 140, and preferably on a portion or a surface of the probe tip 140 that is designed for not having direct contact to the tooth, which is the second surface 142 of the probe tip 140, which is directed away from the surface of the tooth 120. While the first surface 141 is facing the surface of the tooth 120, the second surface 142 is facing in the other direction and preferably in the direction of the optical sensor 150.

Thus, the optical sensor 150, which is directed towards the second surface 142 of the probe tip 140, can detect light 171 that is reflected and/or emitted from the second surface 142 of the probe tip 140 and can determine a colour or an amount of light 171 reaching the optical sensor from the direction of the second surface 142 of the probe tip 140. In particular, the optical sensor 150 can be configured for detecting a change in the amount of light 171 reaching the optical sensor 150 from the second surface 142 of the probe tip 140. Thus, an increasing amount of plaque 110 deposited on the second surface 142 of the probe tip 140 changes an optical property like a reflectivity, a fluorescence or a luminescence of the second surface 142 and results in a change of the amount of light 171 that is reaching the optical sensor 150.

In order to detect plaque 110 optically, the mechanical probe 130 and in particular the second surface 142 is preferably dark colored, e.g. black. By choosing a dark probe material the moving plaque layer changes the lightness of the probe which can be optically measured using an optical sensor 150 or a camera. As the probe tip moves through the plaque 110, at least a part of the second surface 142 is shielded by the whitish plaque that is removed from the surface of the tooth 120. This shielding can be dynamically monitored using the optical sensor 150 like a video camera. An increase or a variation in light 171 coming back from the second surface 142 is used to generate a positive signal that plaque 110 is present on the probe tip and therefore on the surface of the tooth 120. Alternatively, the mechanical probe 130 and in particular the second surface 142 can be translucent and illuminated from the inside of the probe tip. Thus, light emitted from the second surface can be at least partially shielded or absorbed by plaque on the second surface, thus reducing the amount of light reaching the optical sensor.

The processing unit 160 is configured for receiving a signal 151 of the optical sensor 150 that is indicative of the light 171 that is reflected and/or emitted from the second surface 142 of the probe tip 140. Thus, the presence of plaque 110 on the surface of the tooth 120 is determined based on a variation of the light 171 that is reflected and/or emitted from the second surface 142 of the probe tip 140. Thus, preferably, the presence of plaque 110 on the surface of the tooth 120 can be derived from the presence of plaque 110 on the second surface 142 of the probe tip 140 when the probe tip 140 is being moved along the surface of the tooth 120 and loosens plaque 110 from the tooth that is deposited on the probe tip 140. The processing unit 160 may also be trained using artificial intelligence tools to recognize the presence or absence of plaque 110 from generated optical signals or images.

The mechanical probe 130 can be extending from a handle, a device head like brush head or a nozzle, or a body 101 of the plaque detection device 100 towards the surface of the tooth 120, and can have a connecting element 135 connecting the probe tip 140 with a specifically designed tip shape to the body 101 of the plaque detection device 100. Preferably, the connecting element 135 is not fully rigid but may able to bend up and down, following the geometry of the teeth, and keeping contact with the tooth surface, similar to a brushing bristle. In addition, the connecting element 135 of the mechanical probe 130 is preferably not perpendicular to the handle or body 101 but is angled relative to a longitudinal axis 102 of the plaque detection device 100. Thus, bending of the mechanical probe 130 as such and therefore contour following is facilitated. Another advantage of the angulation may be that the optical sensor 150 can image the probe tip 140 from the top.

The probe tip 140 can be shaped such that at least a part of the probe tip penetrates an interspace between the surface of the tooth 120 and a layer of the plaque on the tooth, and removes at least a part of the plaque 110 from the tooth by depositing the removed plaque on the probe tip 140. For example, the probe tip 140 can have a V-shaped edge between the first surface 141 and the second surface 142 to lift plaque 110 over the edge of the probe tip 140.

With continuing reference to Fig. 2, the plaque detection device 100 can optionally comprise an illumination source 170 for illuminating the second surface 142 of the probe tip 140 with light 171. This may enhance a homogenous illumination of the second surface 142 of the probe tip 140 and ensure a constant illumination of the probe tip 140, even in dark environments like the mouth of a user. Alternatively, the probe can be light-emitting, such that the light source is arranged within the probe, e.g. the probe can be a light guiding probe, configured to emit the light at the second surface. The illumination source may thus be in a handle of the plaque detecting device 100, and the light may be transported through a light guide to the probe tip, or the illumination source may be arranged at or in the probe tip. Alternatively, the probe may be electrically wired and can have a tiny illumination source directly located at the second surface. If plaque is attaching to or moving over the second surface, the light will be partially blocked and the light intensity measured by the optical sensor will change, which is indicating plaque present on the second surface.

Optionally, the plaque detection device 100 can comprise a removing system 180 for removing plaque 110 sticking to the second surface 142, and/or the second surface 142 of the probe tip 140 can be configured for minimizing an amount of plaque 110 sticking to the second surface 142. As the plaque detection device 100 may suffer from the plaque 110 sticking to the probe tip 140, reducing the sensitivity over the course of the application, the plaque detection device 100 can provide a fluidic jet for removing the plaque 110 from the second surface 142 of the probe tip, or can utilize vibrations for shaking of the plaque from the probe tip 140. In addition or alternatively, the second surface 142 of the probe tip 140 can have a very smooth and slippery surface for avoiding plaque sticking to the second surface 142. When new plaque 110 comes in the lightness of the probe, the plaque will still pile up and decrease as parts of the plaque 110 are gliding or falling off. This varying signal can be an indication that plaque 110 is present on a certain location of the teeth. The use of non-sticking probe materials like for example polytetrafluoroethylene can additionally minimize plaque 110 sticking to the probe tip 140. A solution to sticking plaque 110 can also be to remove the plaque 110 intermittently from the probe tip 140. This may be done, for example, with certain vibrations of the probe tip that can shake the plaque off, or with fluidic jets. If the plaque detection device 100 is integrated in an oral irrigator, the fluidic jet may efficiently be provided, and the jet pulses can be used to remove sticking plaque so the probe is clean when ploughing into the next piece of plaque layer.

Further, the plaque detection device 100 can optionally comprise a position sensor 190 that is configured for determining a position of the plaque detection device 100 with respect to a mouth of a user. Thus, the processing unit 160 can be configured for providing a position of the plaque 110 on the surface of the tooth 120 with respect to the mouth of the user. Thus, the plaque detection device 100 can provide a map of the mouth of the user and indicate in which regions of the mouth the teeth are still covered with plaque 110 and should be brushed more carefully.

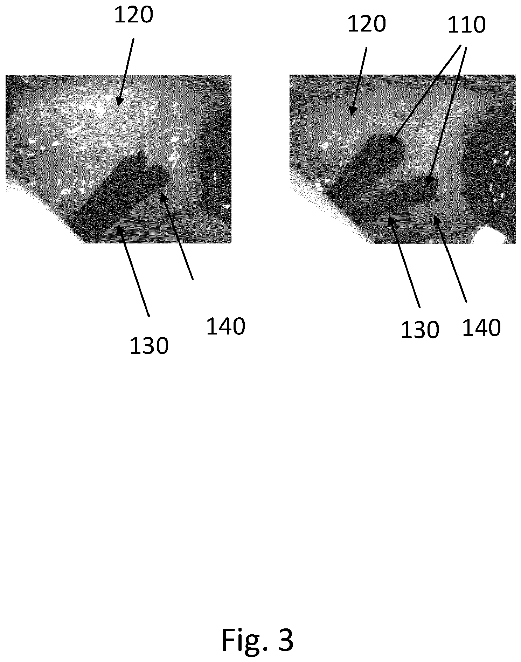

Fig. 3 shows a photograph of plaque 110 on a probe tip 140 of a plaque detection device 100 according to an embodiment of the invention. The mechanical probe 130 may be simply a standard shape black brushing bristle. Alternatively, the plaque detection device 100 can comprise a plurality of mechanical probes 130. In this example, bristles of a tooth brush are used as mechanical probe 130. To improve sensitivity, the probe tip 140 may have a specific shape, e.g. a thin flat shape or a V-shape like a snow plough, as such shapes more easily plough below the plaque layers. This embodiment was tested in the laboratory. An oral biofilm was cultured on a surface of a tooth 120 previously extracted. A bristle tuft was colored black, which is shown in Fig. 3. The left photograph of Fig. 3 shows a clean surface of a tooth 120 with the black bristles as mechanical probe 130 with the probe tip 140. The right photograph of Fig. 3 shows a biofilm covering the surface of the tooth 120 after moving the bristles through the biofilm. The loosened biofilm covers the black bristle tips, giving them a lighter shade, which could be picked up using image analysis by the processing unit 160 or a trained artificial intelligence algorithm. As can be seen in the right photograph of Fig. 3, a slight layer of plaque 110 is covering the probe tip 140 of the mechanical probe 130.

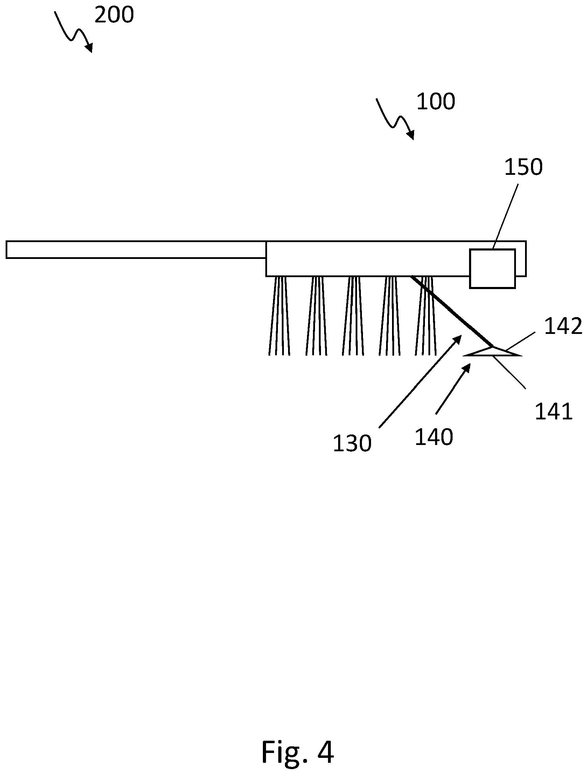

Fig. 4 shows a schematic setup of an oral care apparatus 200 comprising the plaque detection device 100 for the detection of plaque 110 on a surface of a tooth 120 according to an embodiment of the invention. Thus, dental plaque can detected using a mechanical probe 130 and optical sensing of the interaction of plaque 110 with the first surface 141 and the second surface 142 of the probe tip 140 using an optical sensor 150. Various embodiments can be envisioned: The plaque detection device 100 can be a dedicated plaque sensing tool, or it can be integrated in an oral care apparatus 200 such as a toothbrush, an oral irrigator or a brushing mouthpiece. The key parts of the invention are the mechanical probe 130 and the optical sensor 150 with the processing unit 160 for analysis of the detected signals.

For example, the mechanical probe 130 can have a round probe tip, or it can be an elongated probe with at least a round or oval cross section, like, e.g. a bristle of a tooth brush. In this case, the first surface and the second surface are essentially both part of the same outside surface of the cylindrical probe, with one side of the probe tip contacting the tooth and the other opposite side of the probe being directed away from the tooth. As another example, the probe tip 140 can have a basically flat outer shape, with a lower surface, the first surface 141, contacting the tooth, and an upper surface, the second surface 142, being positioned essentially opposite to the first surface. In this case, the first surface and the second surface can be separated from each other by more or less sharp edges of the probe tip 140. Optionally, the second surface can be directed away from the tooth at a certain angle, that may be, for example, 45 degrees with respect to the surface of the tooth. This may be in particular useful if the optical sensor 150 is positioned not directly above the probe tip 140 with respect to a contact point of the probe tip 140 with the tooth, but slightly further away to the side in a direction parallel to the surface of the tooth 120.

While the standard bristle tip configuration can detect thicker plaque layers, at locations with a thinner plaque layer, a not sufficient amount of plaque may be moved over the bristles as second surface 142 to detect a signal. Thinner bristles may improve plaque detection, though they may become too soft at some point, unless they are in fact very short thin bristles mounted on a thicker connecting element. Other tip geometries to enable a higher sensitivity may be thin sheets or wedge shape elements. The wedge shape may in the other dimension also form a V-shape, similar to a snow plough. Such a shape may have the further advantage that new plaque layers push plaque which sticks to the probe away, showing dynamic fluctuations of the lightness which could be another signal used for detecting plaque. Of course, for comfort for the user of the plaque detection device, the probe tip 140 should not be too sharp.

With respect to the optical sensor 150, this can be a camera. Color is not required and also a high resolution may not be necessary. A camera in combination with multiple mechanical probes 130, for example a line of probes, would even allow for plaque mapping on a tooth. As the user is moving the system over the teeth plaque data would come back at different heights on the tooth, and when combined with location sensing a full mouth plaque map may be created. The camera may also recognize the gum line and map the plaque signals with respect to the gum line. Less advanced but cheaper embodiments may be made using a simple optical sensor. This sensor can detect average changes in the light 171 coming back from a probe or the probes, in this way detecting average plaque levels on a certain tooth. For robust optical sensing, the device can be supplied with a stable illumination source 170, e.g. an LED. Specific wavelengths may be chosen to improve contrast between plaque 110 and the probe tip 140 or between tooth and gum surfaces.

A potential problematic issue may be that toothpaste used in tooth brushing, can block the optical path and disturb the detection mechanism of the optical sensor. Here additional measures may need to be performed to move toothpaste out of the region of the probe tip, which can be, for example, toothpaste wipers. Again, water or air jets can be a beneficial solution to remove the blocking toothpaste, in particular if a toothbrush with an integrated interdental cleaning jet is used. Alternatively, the optical sensor 150 can only uses images where toothpaste is temporarily out of the field of view of the optical sensor. As the brush is moving, the toothpaste gets moved around by the bristles and regularly local toothpaste-free spots can appear due to this mixing around. Another strategy may be to use the toothbrush comprising the plaque detection device 100 once in a while without toothpaste in order for detecting plaque. Getting this plaque removal feedback once a week may for example be sufficient to enhance the brushing technique and improve the oral health outcome. If the plaque detection device 100 is applied in an oral irrigator or in a separate plaque scanning device, the toothpaste problem does not exist. Of particular interest may be a fluorescence camera or scanner to detect possible problematic areas that appear red fluorescent. Red fluorescence can be emitted from aged plaque as well as from tartar and carious lesions. Adding the current plaque detection device 100 to such a scanner would make also the younger plaque visible, which can be very relevant to consumers. The younger plaque still has an auto-fluorescence, not in the red but in a similar emission spectrum as the teeth. Therefore, using the typical violet excitation, both the young plaque and the teeth have a green fluorescence and there is no contrast to see the plaque. Using the present invention with the mechanical probe, the green fluorescent plaque can be seen moving over the probe, or the probe moving under the young green fluorescent plaque. The probe material should not be fluorescent by itself, or should have a different emission spectrum to give contrast. The probe can in this concept also help in differentiating the different sources of red fluorescence identified with this scanner. If the source is old plaque it will also be seen that the probe is digging in this old plaque layer, but if it is tartar or caries the probe will simply move over the spot as there is no soft plaque layer there.

In an embodiment detecting fluorescence of the plaque, a specific wavelength range of the excitation light of the illumination source illuminating the probe tip and the plaque may be required as well as an optical filter in front of the optical sensor to filter out those light excitation wavelengths, only capturing the emitted wavelengths of the fluorescence. For example, for detecting the auto-fluorescence of plaque, often violet to blue excitation wavelengths are chosen in the range of 400 to 480nm, as the plaque generally shows a greenish fluorescence at those excitation wavelengths. Old plaque will also have red fluorescence, which is strongest around 400 to 420nm excitation. Going below 400nm is possible, but less preferred as ultra violet light may be harmful to the oral tissues. The probe properties for this mode are preferably non-fluorescent. In the fluorescence image a non-fluorescent probe will appear black, and plaque moving over the second surface of the probe will thus make that surface appear brighter, very similar to the reflection embodiment with a black probe. The probe may also have a different fluorescence, e.g. low or very high brightness or different color, than the auto-fluorescence of plaque.

The employed sensing mechanism of the plaque detection device of the present invention of course also removes the plaque partially or even fully when detecting it, so additional cleaning may in fact not be required anymore if the user gets the signal that plaque is or was present. Still, it would be very useful to test regularly with such a device whether an oral hygiene procedure was successful used and whether there are locations which are consistently poorly brushed. With this information users can be coached to brush certain locations better or longer.

The device according to the present invention may also be used in other personal health applications, for example looking at skin properties, and more specifically looking at deposits or loose material on the skin such as sebum or dead skin cells. This may be applied in exfoliation, acne assessments or shaving. Detecting deposits on other body parts, such as skin could also be of general interest to personal health based on imaging in personal health devices. On the skin, deposits may be found of sebum, which can be relevant for acne, or dead skin cells that are relevant for exfoliation.

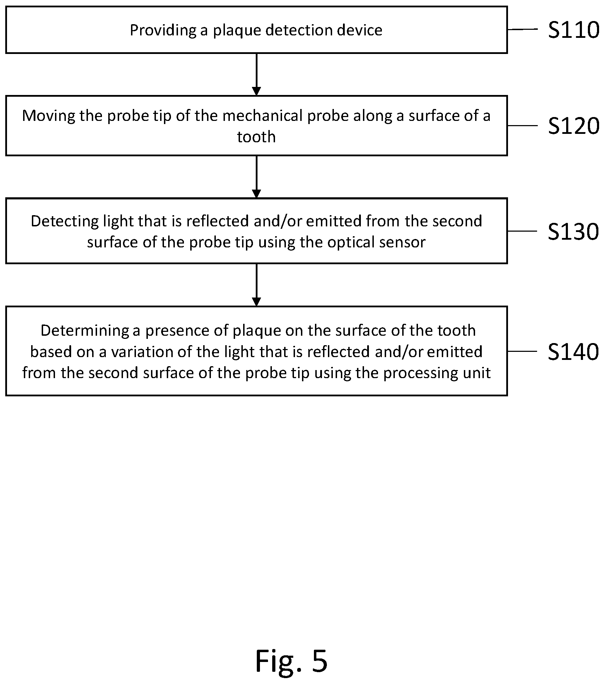

Fig. 5 shows a block diagram of a method for the detection of plaque 110 on a surface of a tooth 120 using the plaque detection device 100 according to an embodiment of the invention. The method comprises the step S 110 of providing a plaque detection device according to any of the preceding embodiments, and the step S 120 of moving the probe tip of the mechanical probe along a surface of a tooth. The method comprises further the step S 130 of detecting light that is reflected and/or emitted from the second surface of the probe tip using the optical sensor, and the step S 140 of determining a presence of plaque on the surface of the tooth based on a variation of the light that is reflected and/or emitted from the second surface of the probe tip using the processing unit. Optionally, the method comprises the step of scraping plaque off the surface of the tooth and depositing the scraped off plaque on the second surface of the probe tip.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: plaque detection device
- 101: body
- 102: longitudinal axis
- 110: plaque
- 120: surface of a tooth
- 130: mechanical probe
- 135: connecting element
- 140: probe tip
- 141: first surface
- 142: second surface
- 150: optical sensor
- 151: signal
- 160: processing unit
- 170: illumination source
- 171: light
- 180: removing system
- 190: position sensor
- 200: oral care apparatus

## Claims

1. A plaque detection device (100) for the detection of plaque (110) on a surface of a tooth (120), the plaque detection device (100) comprising:
a mechanical probe (130) comprising a probe tip (140) being configured for moving along a surface of a tooth (120), wherein the probe tip (140) comprises a first surface (141) and a second surface (142), the first surface (141) being configured for moving along the surface of the tooth (120), and the second surface (142) being directed away from the surface of the tooth (120);
an optical sensor (150), the optical sensor (150) being directed towards the second surface (142) of the probe tip (140) and configured for detecting light (171) that is reflected and/or emitted from the second surface (142) of the probe tip (140); and
a processing unit (160), the processing unit (160) being configured for receiving a signal (151) of the optical sensor (150) indicative of the light (171) that is reflected and/or emitted from the second surface (142) of the probe tip (140), and configured for determining a presence of plaque (110) on the surface of the tooth (120) based on a variation of the light (171) that is reflected and/or emitted from the second surface (142) of the probe tip (140).

2. The plaque detection device (100) according to claim 1, wherein the presence of plaque (110) on the surface of the tooth (120) is determined by the presence of plaque (110) on the second surface (142) of the probe tip (140) when the probe tip (140) is being moved along the surface of the tooth (120).

3. The plaque detection device (100) according to any of the preceding claims, wherein the probe tip (140) is configured for mechanically scraping plaque (110) off the surface of the tooth (120) and depositing the scraped off plaque (110) on the second surface (142) of the probe tip (140).

4. The plaque detection device (100) according to any of the preceding claims, wherein the second surface (142) of the probe tip (140) comprises an optical property that is different from an optical property of plaque.

5. The plaque detection device (100) according to any of the preceding claims, wherein the second surface (142) of the probe tip (140) is colored black.

6. The plaque detection device (100) according to any of the preceding claims, further comprising an illumination source (170) configured for illuminating the second surface (142) of the probe tip (140) with light (171).

7. The plaque detection device (100) according to any of the preceding claims, wherein the optical sensor (150) is a camera.

8. The plaque detection device (100) according to any of the preceding claims, wherein the plaque detection device (100) comprises a plurality of mechanical probes (130).

9. The plaque detection device (100) according to any of the preceding claims, wherein the mechanical probe (130) comprises a connecting element (135) configured for connecting the probe tip (140) to a body (101) of the plaque detection device (100).

10. The plaque detection device (100) according to claim 9, wherein the connecting element (135) is flexible, and/or wherein the connecting element (135) is angled with respect to a longitudinal axis (102) of the plaque detection device (100).

11. The plaque detection device (100) according to any of the preceding claims, wherein the plaque detection device (100) comprises a removing system (180) configured for removing plaque (110) sticking to the second surface (142), and/or wherein the second surface (142) of the probe tip (140) is configured for minimizing an amount of plaque (110) sticking to the second surface (142).

12. The plaque detection device (100) according to any of the preceding claims, wherein the plaque detection device (100) further comprises a position sensor (190) configured for determining a position of the plaque detection device (100) with respect to a mouth of a user, and wherein the processing unit (160) is configured for providing a position of the plaque (110) on the surface of the tooth (120) with respect to the mouth of the user.

13. An oral care apparatus (200) comprising the plaque detection device (100) according to any of claims 1 to 12.

14. A method for the detection of plaque (110) on a surface of a tooth (120) using the plaque detection device (100) according to any of claims 1 to 12, the method comprising the steps of:
providing (S110) a plaque detection device (100) according to any of claims 1 to 12;
moving (S120) the probe tip (140) of the mechanical probe (130) along a surface of a tooth (120);
detecting (S130) light (171) that is reflected and/or emitted from the second surface (142) of the probe tip (140) using the optical sensor (150); and
determining (S140) a presence of plaque (110) on the surface of the tooth (120) based on a variation of the light (171) that is reflected and/or emitted from the second surface (142) of the probe tip (140) using the processing unit (160).

15. The method according to claim 14, further comprising the step of scraping plaque (110) off the surface of the tooth (120) and depositing the scraped off plaque (110) on the second surface (142) of the probe tip (140).
